(19) Europäisches Patentamt
European Patent Office
Office européen des brevets

(11) **EP 4 386 380 A1**

(12) **EUROPEAN PATENT APPLICATION**

(43) Date of publication:
**19.06.2024 Bulletin 2024/25**

(21) Application number: **23215950.9**

(22) Date of filing: **12.12.2023**

(51) International Patent Classification (IPC):
***G01N 33/38*** *(2006.01)*

(52) Cooperative Patent Classification (CPC):
**G01N 33/383**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.12.2022 TR 202219072**

(71) Applicant: **Fibrobeton Yapi Elemanlari Sanayi Insaat Ve**
**Ticaret Anonim Sirketi**
**34810 Istanbul (TR)**

(72) Inventors:
• **MARASLI, MUHAMMED**
  **34810 Istanbul (TR)**

• **SUBASI, SERKAN**
  **81620 Düzce (TR)**
• **GUNTEPE, SEFA**
  **34810 Istanbul (TR)**
• **CEVIRMEN, MERT**
  **34810 Istanbul (TR)**
• **OZDAL, VOLKAN**
  **34810 Istanbul (TR)**

(74) Representative: **Yalvaç, Oya**
**Deris Patent ve Marka Acentaligi A.S.**
**Inebolu Sokak, No: 5**
**Deris Patent Binasi**
**Kabatas/Setüstü**
**34427 Karakoy/Istanbul (TR)**

(54) **MATURITY MEASUREMENT METHOD AND MATURITY MEASUREMENT DEVICE FOR GLASS FIBER REINFORCED CONCRETES**

(57) The invention discloses a maturity measurement device, a maturity measurement system and a maturity measurement method for converting a recorded temperature history of a glass fiber reinforced concrete into compressive/ flexural strength, allowing accurate and instantaneous estimation of concrete strength at a desired age.

FIGURE 1

**Description**

**Technical Field**

The present invention relates to a maturity measurement method for measuring the maturity of a glass fiber reinforced concretes and for analyzing such data, and to a maturity measurement device for utilizing this method.

**Background**

Concrete is a widely used building material. In general, the "mass concrete" structures, i.e. concrete structures which are relatively larger than the rest, tend to display a higher concrete temperature following placement or deposition thereof compared to that of any other ordinary concrete structures. This tendency is more pronounced in the interior of such concrete structures. Consequently, in such mass concrete structures, the strength development of concrete at early ages is very high, and accordingly, due to temperature variations within the interior of concrete or to temperature differences between the environment and the concrete, undesirable events like cracking occur, resulting in an adverse effect on the strength of concrete. Air and water content represent the two components of concrete mix which have a substantial influence on the ultimate strength of concrete and can vary significantly, even over a particular period of time, from batch to batch, day to day and week to week. Accordingly, the state of strength development of concrete should be properly estimated and controlled by the builders. In the prior art, the durability of cement-based materials is estimated based on their temperature history. Temperature values are converted into maturity values by means of time-dependent functions. In current applications where more advanced measurement methods are used, the maturity method is used as the measurement method. In such applications, the maturity value is converted into compressive strength. Two separate methods are used to calculate the maturity index from the measured temperature history of concrete. One of these methods is the method known as Nurse-Seul, which uses the same maturity function for both normal and customized concretes (concretes in which variables such as the fiber condition of the concrete, the fiber ratio or the special binders used are controlled), focusing on the initial temperature rather than the nature of the binder used:

$$M = \sum_{i=0}^{t} (T - T_0)\Delta t$$

where M is maturity at age t, and uses the parameters, including the average concrete temperature (T) over a time interval, the reference temperature ($T_0$) and the time interval ($\Delta$t).

Another maturity measurement method is the De Vree weighted maturity measurement method:

$$\Delta M_W = 10\left[C^{(0,1T-1,245)} - C^{(-2,245)}\right]/\ln C$$

where C is a coefficient of the cement.

Flexural strength is a critical parameter for more accurate estimation of concrete strength in glass fiber reinforced concretes. However, in the state of the art, there is no device or measurement method that takes into account the correlation between flexural strength and maturity.

Also, in the state of the art, such measurements are often utilized to track demolding time of normal site concretes. Since the devices carrying out said measurements are used in medium and long-term examinations, temperature and maturity measurements are calculated in such devices at fixed intervals. In other words, in the state of the art, no warning is triggered when a desired maturity value is reached or when the temperature increases. And, this causes problems in the stage of production planning in such areas where a mass production is carried out on a frequent basis.

As can be appreciated when the presence of such shortcomings in the state of the art is considered, there is a need for a maturity measurement device, a maturity measurement system and a maturity measurement method that allow measuring the strength of glass fiber reinforced concretes at desired time periods, and provide more precise and healthy measurement values as to the strength of glass fiber reinforced concretes.

**Summary of the Invention**

The present invention is provided as a solution to the problems of the prior art described in detail above.

An objective of the present invention is to realize a maturity measurement device, a maturity measurement system, and a maturity measurement method, for enabling in-situ real-time monitoring of the temperature of a glass fiber reinforced concrete and in-situ estimation of the compressive and flexural strength of the glass fiber reinforced concrete by using the maturity method.

Another objective of the present invention is to realize a maturity measurement device, a maturity measurement system, and a maturity measurement method, for providing parameters such as temperature, maturity, strength and an estimated dismantling time to a user at the desired time intervals.

Another objective of the present invention is to realize a maturity measurement device, a maturity measurement system, and a maturity measurement method, for providing an estimate of the flexural strength of a concrete, particularly of a fibrous concrete, to the user in a non-destructive manner.

Another objective of the present invention is to realize a maturity measurement device, a maturity measurement system, and a maturity measurement method, which have an audible or alerting warning feature for warning the user when a desired maturity value is reached or when the temperature rises.

**Detailed Description of the Invention**

The present invention relates to a maturity measurement method, a maturity measurement system, and a maturity measuring device, for measuring the maturity of a glass fiber reinforced concrete based on flexural and compressive strength results.

As used herein, "flexural strength" (also known as bending strength, or modulus of rupture) is a measure of the tensile strength of the outer fiber of a cement product. This property is determined by means of an apparatus which applies loads to cylindrical or prismatic specimens at three or four points until the respective material breaks.

As used herein, "compressive strength" is defined as the resistance of a concrete material against compressive loads to which it is exposed in its axial direction. In other words, it is defined as the highest stress that develops in a concrete structure being subjected to a compressive load in its axial direction.

As used herein, the maturity method is a non-destructive method for estimating the compressive and flexural strength of a cement-based material at different time intervals. This maturity method provides a reliable estimate of the future strength of a placed concrete based on the results obtained by monitoring the early strength development of the same. For a good concrete durability, the concrete should not gain strength too fast. Therefore, such monitoring is necessary to improve the quality of concrete.

In the prior art, a recorded temperature history of a concrete is used to calculate an estimate of concrete strength by applying one or more industry standard formulas. On the other hand, the novel maturity method according to the present invention provides an accurate prediction of concrete strength by converting a measured concrete temperature value into the flexural and compressive strength.

A concrete maturity measurement method of the present invention comprises the steps of:

    a) disposing at least one temperature sensor in a glass fiber reinforced concrete,

    b) measuring the temperature of the glass fiber reinforced concrete by means of the temperature sensor, and transmitting the obtained temperature data to a maturity measurement device in communication with the temperature sensor, and

    c) determining the maturity, flexural strength and compressive strength of the glass fiber reinforced concrete by the interface in the maturity measurement device based on the temperature data obtained from the glass fiber reinforced concrete,

the method is characterized in that the C value of the concrete, and the maturity-strength change formula are pre-calculated and entered into the interface in order to carry out the calculation of the maturity, flexural strength and compressive strength values of the glass fiber reinforced concrete based on the temperature data.

The coefficient C, and the maturity-strength change formula, which are used in the method, are obtained by means of a method including the steps of:

    i) placing the glass fiber reinforced concrete specimens in reservoirs containing water at a constant temperature,

    ii) subjecting the glass fiber reinforced concrete specimens kept in the reservoirs to compressive/flexural strength tests at specified time intervals, and recording the temperature and time data as well as the compressive and flexural strength values obtained in said tests,

    iii) plugging each of the strength values obtained in the step ii), together with the temperature and time values, into the equation in the De Vree maturity model, and calculating the maturity values based on at least 2 C values ranging from 1 to 2,

    iv) calculating the correlation between the maturity values calculated in the step iii),

v) selecting the highest correlation value, and determining the C value plugged into the equation in the De Vree maturity model to achieve this correlation value,

vi) subjecting the glass fiber reinforced concrete specimens to compressive/flexural strength tests,

vii) obtaining the maturity values by using the determined C value, together with the time and temperature values determined for calibration purposes, in the model,

viii) carrying out an analysis by using the obtained maturity values, together with the strength (compressive/flexural) values determined for calibration purposes, to obtain a maturity-strength change formula.

As there are two or more quantitative variables between strength and maturity values, a linear regression calculation is performed. In these calculations, a coefficient of determination is also used to calculate the accuracy of the resulting strength-maturity formula.

For certain isothermal strength tests in the De Vree maturity model mentioned in the standards in accordance with the present invention, it is necessary to calculate the coefficient C, a cement-specific constant where the weighted maturity curves overlap each other.

In a preferred embodiment of the present invention, the glass fiber reinforced concrete specimens of the step i) are produced from the unset concrete. In addition, said concrete specimens are either cylindrical or cubic, preferably cylindrical with a length/diameter ratio of 2.

Said glass fiber reinforced concrete specimens are produced to determine the maturity measurement method. The concrete to be tested is produced with the same unit formula and production method as the concrete for which the maturity measurement method is to be developed, and the concrete specimens are obtained from that concrete. Thereafter, the maturity value and method determined here will be used for the concrete produced with the same unit formula and method.

According to the present invention, in the step i) the glass fiber reinforced concrete specimens are kept in a reservoir at a temperature ranging from 10°C to 100°C and preferably from 20°C to 70°C. In a more preferred embodiment of the present invention, the concrete specimens are divided into separate reservoirs of at least two different temperatures. For example, 6 of the 12 concrete specimens are kept in a reservoir at one temperature, while the other 6 are kept in another reservoir at another temperature. Temperatures here are preferably 20°C and 65°C. Keeping the temperature constant is crucial for the inventive method to give accurate results. Therefore, as long as tests and measurements are in progress, the temperature is measured with instruments available in the state of the art.

According to the present invention, in the step ii) the glass fiber reinforced concretes are subjected to compressive and/or flexural strength tests at intervals of 1 hour or 2 hours. Said tests are conventional tests available in the state of the art, and are preferably compressive/bending strength tests according to EN 12390-3-5 or EN 196-1 standards, and according to TS 13508 and NEN 5970 maturity method calculation standards. Further, as noted, such tests are carried out to find the maturity value calculated in order to obtain the maturity-strength change formula.

In a preferred embodiment of the present invention, the number of the C values in the step iii) is at least 10, each of which ranges from 1 to 2, with an inter-C value difference ranging from 0.01 to 0.1. For example, the first C value may be 1.01, whereas the last C value may be 1.10; and each C value may increase by a difference of 0.01.

The equation in De Vree maturity model according to the present invention is as follows:

$$\Delta M_W = 10\left[C^{(0,1T-1,245)} - C^{(-2,245)}\right] / \ln C$$

In a preferred embodiment of the present invention, the glass fiber reinforced concrete specimens of the step vi) are different from the concrete specimens of the step i), even though both groups of specimens are obtained with the same unit formula and production method.

According to the present invention, the compressive/flexural strength tests in the step vi) are performed according to EN 12390-3-5 or EN 196-1 standards. A regression analysis is performed using the obtained maturity values and the calibrated strength (compressive/flexural) data, and a formula of transition from maturity to strength is determined. This formula is different for each concrete type, fiber type, and fiber amount. By using this method, the C value and the maturity-strength change formula according to a plurality of concrete types, different fiber rates, or different fiber lengths can be obtained.

With said method, the C value and the maturity-strength change formula are obtained. The C value and the change formula are entered into the interface within the device. A temperature sensor is then disposed in the concrete of which maturity, flexural strength, and compressive strength will be determined. The temperature values measured by this temperature sensor are converted into values for the maturity, flexural strength and compressive strength of the concrete by using the determined C value and change formula. Thus, with the maturity measurement device according to the present invention, the maturity, flexural strength and compressive strength values of the concrete are obtained by measuring only the temperature.

In a preferred embodiment of the present invention, the maturity measurement method for the glass fiber reinforced concrete further comprises the process step of transmitting the data obtained by the maturity measurement device to a cloud-based server.

In a glass fiber reinforced concrete maturity measurement method according to the present invention, measurements for medium or long (7-28 days) term use are taken at periodic times, such as at 1, 4, 8, 12, or 18 hours.

In a glass fiber reinforced concrete maturity measurement method according to the present invention, measurements for short term use (1-7 days) are taken at periodic times, such as at 1, 4, 8, 12, or 18 hours. A glass fiber reinforced concrete maturity measurement method according to the present invention comprises the process step of notifying a user with a visual or audible warning when a desired predetermined threshold is reached in at least one of the parameters temperature, flexural strength, compressive strength, and concrete maturity.

A glass fiber reinforced concrete maturity measurement method according to the present invention comprises the process step of determining critical thresholds corresponding to high and/or low values by the user.

A glass fiber reinforced concrete maturity measurement method according to the present invention comprises the process step of taking measurements by the sensor over a measurement period of at least 100 ms.

In a preferred embodiment of the invention, the maturity measurement device further comprises a warning unit for providing an audible or visual warning to alert a user that a particular section of a glass fiber reinforced concrete has reached a certain compressive strength, flexural strength, or temperature.

Throughout the detailed description of the invention, the term "system" is used to describe the sum total of the particular devices, method steps, and interfaces disclosed herein.

In one embodiment of the invention, the maturity measurement system comprises at least one maturity measurement device, and at least one temperature sensor in communication with the device. Said sensor is in communication with the maturity measurement device via a communication unit. The maturity measurement device is configured to transmit the data received from the sensor to a cloud-based server, preferably via a base station. The cloud-based server allows any internet-enabled device, such as smartphones, tablets or computers, to access said data.

In one embodiment of the present invention, maturity data over time is collected by the maturity measurement device and can be displayed via a display of the device. Said data help to understand how fast or slow the concrete is curing.

In the preferred embodiment of the invention, at least one temperature sensor is used to perform real-time temperature measurement of the glass fiber reinforced concrete. According to the invention, at least one sensor is embedded inside the concrete up to the center of the concrete thickness for taking at least one temperature measurement to obtain an estimate of the maturity of the concrete during the early stage curing process.

In one embodiment of the invention, the sensors are connected to the maturity measurement device via a communication unit selected from bluetooth, wireless, and cable.

In the preferred embodiment of the invention, at least one sensor is connected to at least one maturity measurement device via a cable, and data from the sensor are transmitted to the maturity measurement device via the cable.

**EXAMPLE**

Determination of Maturity Value C

The concrete intended to be measured is first produced by its own production process in its own mix proportions. The mix design and process in EN 196-1 standard can be used to test the system. According to said standard, 22.2% cement, 66.7% CEN standard sand, and 11.1% water are added.

a) Water and cement are carefully added to a mixing bowl, by avoiding any loss of cement and water.

b) As soon as the water and cement come into contact with each other, a mixer is started at a low speed while starting the timing of the mixing stages. In addition, the time is recorded to the nearest minute as "zero time." After 30 seconds of mixing, all of the sand is continuously added to the bowl within 30 seconds. The mixer is switched to a high speed, and mixing is continued at this speed for 30 seconds. The prepared mortar is then placed in molds designated for compressive/flexural strength.

In order to determine the maturity measurement method for a concrete type with a specific unit formula and a specific production method, the maturity value of that specific concrete should first be determined. This is a one-time test. Thereafter, the maturity value and method determined here will be used for the concrete produced with the same unit formula and method.

The glass fiber reinforced concrete is produced as 12 cylinder specimens with a length/diameter ratio of 2 in a form that can be subjected to strength tests. 6 of the specimens are added to reservoirs at an average temperature of 20°C and the other 6 are added to reservoirs at an average temperature of 65°C. While the temperature is kept constant in these reservoirs, the compressive/flexural strength test is applied to the concrete specimens in said water reservoirs at the times shown in Table 1 according to EN 12390-3-5 or EN 196-1 standards and according to TS 13508 and NEN 5970 maturity method calculation standards. As of the time the concrete starts to set, the compressive/flexural strength of the concrete is measured at certain intervals according to the abovementioned standards. During the strength test, the temperature value is also measured with a suitable device. The time and temperature data, as well as the measured compressive/bending strength values, are entered to the relevant field in the maturity system as shown in Table 1 below.

Coefficient C

| **Temperature and Time Values** | | **Strength Values** |
|---|---|---|
| 20°C Temperature-1 * | 20°C Time-1 * | 20°C Strength-1 * |
| 20°C Temperature-2 * | 20°C Time-2 * | 20°C Strength-2 * |
| 20°C Temperature-3 * | 20°C Time-3 * | 20°C Strength-3 * |
| 20°C Temperature-4 * | 20°C Time-4 * | 20°C Strength-4 * |
| 20°C Temperature-5 * | 20°C Time-5 * | 20°C Strength-5 * |
| 20°C Temperature-6 * | 20°C Time-6 * | 20°C Strength-6 * |
| 65°C Temperature-1 * | 65°C Time-1 * | 65°C Strength-1 * |
| 65°C Temperature-2 * | 65°C Time-2 * | 65°C Strength-2 * |
| 65°C Temperature-3 * | 65°C Time-3 * | 65°C Strength-3 * |
| 65°C Temperature-4 * | 65°C Time-4 * | 65°C Strength-4 * |
| 65°C Temperature-5 * | 65°C Time-5 * | 65°C Strength-5 * |
| 65°C Temperature-6 * | 60°C Time-6 * | 65°C Strength-6 * |
| CONFIRM | CANCEL | CONFIRM | CANCEL |

## Table 1

Table 2

| time | temperature | compressive strength | Weighted Maturity [°Ch] | | | | | | | | | | | |
|---|---|---|---|---|---|---|---|---|---|---|---|---|---|---|
| | | | C value | | | | | | | | | | | |
| [ h ] | [ °C ] | [N/mm$^2$] | 1,01 | 1,02 | 1,03 | 1,04 | 1,05 | 1,06 | 1,07 | 1,08 | 1,09 | 1,10 | 1,11 | 1,12 |
| 18 | 21 | 14,2 | 554 | 550 | 547 | 543 | 540 | 537 | 533 | 530 | 527 | 524 | 521 | 518 |
| 20 | 22 | 27,5 | 636 | 632 | 628 | 624 | 621 | 617 | 614 | 611 | 607 | 604 | 601 | 598 |
| 22 | 22 | 40,2 | 700 | 695 | 691 | 687 | 683 | 679 | 675 | 672 | 668 | 665 | 661 | 658 |
| 41 | 21 | 65,4 | 1262 | 1254 | 1246 | 1238 | 1230 | 1222 | 1215 | 1208 | 1201 | 1194 | 1187 | 1181 |
| 67 | 21 | 74,4 | 2063 | 2049 | 2035 | 2022 | 2010 | 1997 | 1985 | 1973 | 1962 | 1951 | 1940 | 1930 |
| 70 | 21 | 78,6 | 2155 | 2141 | 2127 | 2113 | 2100 | 2087 | 2074 | 2062 | 2050 | 2038 | 2027 | 2016 |
| | | | | | | | | | | | | | | |
| 7 | 64 | 24,6 | 526 | 534 | 542 | 550 | 559 | 568 | 578 | 587 | 597 | 607 | 618 | 629 |
| 9 | 62 | 33,1 | 657 | 666 | 676 | 686 | 696 | 706 | 717 | 728 | 740 | 752 | 764 | 777 |
| 10 | 62 | 43,6 | 730 | 740 | 751 | 762 | 773 | 785 | 797 | 809 | 822 | 835 | 849 | 863 |
| 11 | 62 | 44,3 | 803 | 814 | 826 | 838 | 850 | 863 | 877 | 890 | 904 | 919 | 934 | 949 |
| 14 | 61 | 47,0 | 1007 | 1021 | 1035 | 1050 | 1065 | 1080 | 1096 | 1113 | 1130 | 1147 | 1165 | 1184 |
| 19 | 61 | 64,9 | 1367 | 1385 | 1405 | 1424 | 1445 | 1466 | 1488 | 1510 | 1533 | 1557 | 1581 | 1607 |
| | | r: | 0,923 | 0,926 | 0,929 | 0,931 | 0,934 | 0,935 | 0,937 | 0,938 | 0,939 | 0,940 | 0,939 | 0,939 |

Using the equation in the De Vree weighted maturity method, weighted maturity values are calculated for C values ranging from 1 to 2 and the correlation (r) value is determined. The C value with the highest correlation is automatically selected by the system for the concrete in the example.

Maturity - Strength (Compressive/Flexural) Function Operation

Once the C value is determined, 6 specimens are reproduced, and kept under ambient conditions, for calibration tests. The maturity device and system are initialized when the samples are in their fresh/wet state. Depending on the type of concrete, compressive/flexural strength tests are performed at certain intervals according to EN 12390-3-5 or EN 196-1 standards. When strength tests are performed, the maturity value is entered into Table 3 via the strength and maturity device of the relevant specimen.

## Test Results for Calibration Specimens

**Strength and Maturity Values**

Calibration Strength-1 *          Calibration Maturity-1 *

Calibration Strength-2 *          Calibration Maturity-2 *

Calibration Strength-3 *          Calibration Maturity-3 *

Calibration Strength-4 *          Calibration Maturity-4 *

Calibration Strength-5 *          Calibration Maturity-5 *

Calibration Strength-6 *          Calibration Maturity-6 *

| CONFIRM | CANCEL |

**Table 3**

**Table 4**

| | number of cube samples | 6 | | | | | |
|---|---|---|---|---|---|---|---|
| cube no. | | R (maturity) | X [ log R ] | $x^2$ | Y (Pressure) [N/mm$^2$] | $y^2$ | xy |
| 1 | | 493 | 2,693 | 7,250 | 12,80 | 163.84 | 34,466 |
| 2 | | 549 | 2,739 | 7,503 | 23,50 | 552,25 | 64,371 |
| 3 | | 659 | 2,819 | 7,946 | 43,00 | 1849,00 | 121,213 |
| 4 | | 1316 | 3,119 | 9,729 | 66,00 | 4356,00 | 205,860 |
| 5 | | 2050 | 3,312 | 10,968 | 70,00 | 4900,00 | 231,828 |
| 6 | | 2050 | 3,312 | 10,968 | 70,00 | 4900,00 | 231,828 |

The maturity system obtains a function specific to the concrete typeby measuring the maturity and strength (compressive and flexural) values. This is the function of transition from maturity value to compressive/flexural strength.

Non-destructive Measurement of Compressive/Flexural Strength of Concrete

The temperature sensor of the maturity measurement device is disposed in the concrete to be measured in accordance with the type of concrete the function of which has been determined. The process is started by adding test information through the system. The temperature sensor measures the temperature of the concrete. It is then converted to the maturity value by the system using the measured temperature data. Next, the compressive/flexural strength value is obtained.

**Claims**

1. A maturity measurement method for a glass fiber reinforced concrete, comprising the steps of:

   a) disposing at least one temperature sensor in the concrete,
   b) measuring the temperature of the concrete by the temperature sensor,
   c) transmitting the measured temperature data to a maturity measurement device in communication with the temperature sensor, and
   d) calculating the maturity, flexural strength and compressive strength of the glass fiber reinforced concrete based on the temperature data obtained from the concrete in the maturity measurement device,

   **characterized in that** a C value of the concrete and a maturity-strength change formula are used in the calculation of the step c).

2. A maturity measurement method for a glass fiber reinforced concrete according to claim 1, wherein the C value is determined by a method comprising the steps of:

   i) placing glass fiber reinforced concrete specimens to be measured for maturity in reservoirs containing water at a constant temperature,
   ii) subjecting the glass fiber reinforced concrete specimens kept in the reservoirs to compressive/flexural strength tests at specified time intervals, and recording the temperature and time data as well as the compressive and flexural strength values obtained in said tests,
   iii) plugging each of the strength values obtained in the step ii), together with the temperature and time values, into the equation in the De Vree maturity model, and calculating the maturity values based on at least 2 C values ranging from 1 to 2,
   iv) calculating the correlation between the maturity values calculated in the step iii),
   v) selecting the highest correlation value, and determining the C value plugged into the equation in the De Vree maturity model to achieve this correlation value.

3. A maturity measurement method for a glass fiber reinforced concrete according to claim 2, wherein in the step i) the reservoirs containing the glass fiber reinforced concretes to be measured for maturity are of two types and at two different temperatures.

4. A maturity measurement method for a glass fiber reinforced concrete according to claim 3, wherein the reservoir temperature values are 20°C and 65°C.

5. A maturity measurement method for a glass fiber reinforced concrete according to any one of claims 2 to 4, wherein a difference ranging from 0.01 to 0.1 exists between the C values in the step iii).

6. A maturity measurement method for a glass fiber reinforced concrete according to any one of claims 2 to 5, wherein the equation in the De Vree maturity model is as follows:

$$\Delta M_W = 10 \left[ C^{(0,1T-1,245)} - C^{(-2,245)} \right] / \ln C$$

7. A maturity measurement method for a glass fiber reinforced concrete according to any one of the preceding claims, wherein the maturity-strength change formula is determined by a method comprising the steps of:

i) subjecting the glass fiber reinforced concrete specimens to compressive/flexural strength tests, and obtaining the compressive and flexural strength values,
ii) obtaining the maturity values, based on the determined C value of the glass fiber reinforced concrete, as well as the time and temperature data,
iii) carrying out an analysis by using the compressive and flexural strength values and the maturity values, and determining a change formula.

8. A maturity measurement method for a glass fiber reinforced concrete according to any one of the preceding claims, further comprising the step of notifying a user with a visual or audible warning when a predetermined threshold is reached in at least one of the parameters temperature, flexural strength, compressive strength, and concrete maturity.

9. A maturity measurement method for a glass fiber reinforced concrete according to any one of the preceding claims, further comprising the step of taking measurements by the sensor over a measurement period of at least 100ms.

10. A maturity measurement device for use in measurement of maturity of a glass fiber reinforced concrete, comprising a temperature sensor and a hardware to which the temperature sensor is connected, **characterized in that** the hardware is configured to be capable of converting temperature data measured by the temperature sensor into the maturity, compressive strength, and/or flexural strength data.

11. A maturity measurement device according to claim 10, further comprising a warning unit for providing an audible or visual warning to alert a user that a particular section of a glass fiber reinforced concrete has reached a certain maturity, compressive strength, flexural strength, or temperature.

Regression-Calibration Line

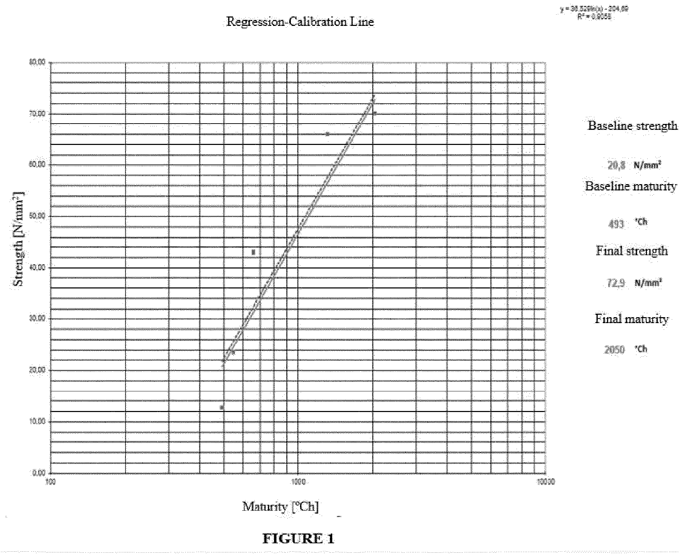

FIGURE 1

Europäisches
Patentamt

European
Patent Office

Office européen
des brevets

# EUROPEAN SEARCH REPORT

Application Number

EP 23 21 5950

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARASLI MUHAMMED ET AL: "Development of a maturity method for GFRC shell concretes with different fiber ratios", EUROPEAN JOURNAL OF ENVIRONMENTAL AND CIVIL ENGINEERING, vol. 26, no. 16, 27 January 2022 (2022-01-27), pages 8458-8476, XP093150813, ISSN: 1964-8189, DOI: 10.1080/19648189.2022.2028190 | 1,7-11 | INV. G01N33/38 |
| Y | * the whole document * | 2-6 | |
| Y | EGMOND BRAM VAN ET AL: "Gewichtete Reife des Betons", CEMENTBULLETIN, vol. 67, no. 11, November 1999 (1999-11), page 3, XP093150793, ISSN: 1422-0717, DOI: 10.5169/seals-153854 * the whole document * | 2-6 | |
| X | Kehl Russel J. ET AL: "Match-Cure and Maturity: Taking Concrete Strength Testing to a Higher Level", , August 1998 (1998-08), pages 1-4, XP093151213, Retrieved from the Internet: URL:https://rosap.ntl.bts.gov/view/dot/14850/dot_14850_DS1.pdf [retrieved on 2024-04-12] * the whole document * | 1,10 | TECHNICAL FIELDS SEARCHED (IPC) G01N |

The present search report has been drawn up for all claims

| Place of search | Date of completion of the search | Examiner |
|---|---|---|
| The Hague | 12 April 2024 | Steinmetz, Johannes |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)